# EUROPEAN PATENT APPLICATION

(11) **EP 4 830 763 A1**
(43) Date of publication of application: **28.10.2026**
(21) Application number: 25172295.5
(22) Date of filing: 24.04.2025
(51) Int. Cl.: A61N 1/05, A61B 5/00, A61N 1/362, A61N 1/372, A61N 1/375, A61N 1/378

(54) **TISSUE INTERFACE AND CARDIAC PACEMAKER**

(71) Applicant: Celtro GmbH, 01099 Dresden (DE)
(72) Inventor: TEEPE, Gerd, 01099 Dresden (DE); THAYYIL, Manu Viswambharan, 01187 Dresden (DE); JORAM, Niko, 01217 Dresden (DE); DRECHSEL, Tom, 01217 Dresden (DE); PIORKOWSKI, Judith, Chanhassen, 55317 (US); PAPE, Forrest, New Brighton, 55112 (US); BELALCAZAR, Hugo Andres, Minneapolis, 55419 (US); BUDNY, Jaroslaw, 51414 Opatija (HR)
(74) Representative: Maikowski & Ninnemann Patentanwälte Partnerschaft mbB

(57) **Abstract**

The instant invention relates to a tissue interface which is adapted to contact with a voltage source from electrochemical potentials inside a tissue comprising at least one microneedle or one microcable wherein a part of the at least one microneedle or the at least one microcable has an isolating layer and that a further part of the at least one microneedle or at least one microcable has a coating. Further a device and a method for signal recording and charge collection from a voltage source comprising the tissue interface are disclosed. Furthermore, a cardiac pacemaker comprising the tissue interface and a method for stimulating myocardial tissue utilizing the cardiac pacemaker are disclosed.

## Description

The instant invention relates to a tissue interface which is adapted to contact with a voltage source from electrochemical potentials inside a tissue comprising at least one microneedle or one microcable wherein a part of the at least one microneedle or the at least one microcable has an isolating layer and that a further part of the at least one microneedle or at least one microcable has a coating. Further a device and a method for signal recording and charge collection from a voltage source comprising the tissue interface are disclosed. Furthermore, a cardiac pacemaker comprising the tissue interface and a method for stimulating myocardial tissue utilizing the cardiac pacemaker are disclosed.

Due to the miniaturization of medical devices, the availability of reliable power sources remains an important technical issue. Batteries are frequently used as power sources. Their size requirements are a fundamental limiting factor when trying to reduce device dimensions. Batteries also need recharging or replacement. In more and more applications recharging or replacement of batteries is clinically, economically and logistically impractical, laborious, or potentially dangerous.

For example, batteries are used as power supply in pacemakers. When the battery runs empty, it must either be recharged or even be surgically replaced. Reported approaches for recharging solutions include for example external energy supplied through external magnetic induction (US 3867950 A1), energy harvested from solar cells (US 2009326597 AA), or energy harvested from cardiac motion [1-4]. Main points of concerns include (i) available energy amounts, (ii) device sizes and implantation complexity, (iii) long-term performance inside a human body, and (iv) associated patient management. Until today none of such recharging/harvesting methods has matured into a clinically approved and widely used cardiac pacemaker.

Therefore, it would be of high interest to provide a power supply for such applications which would allow for energy autonomy, very small device sizes, ease of implantation, conduction system pacing capabilities, easy retrieval options, no lead requirements, and the ability to integrate energy supply with other electrical device functionalities (e.g. sensing and pacing).

The basic principle for collecting electrical energy directly from tiny sources like biological cells is known.

WO 2021/089530 A1 discloses a device for collection of tiny charges in the Nano-Coulomb-range comprising at least one capacitor stack. The capacitor stack comprises n capacitors, which are charged one after the other by tiny charges. After charging all n capacitors of the capacitor stack, all n capacitors are discharged into a further capacitor outside the capacitor stack at once. Afterwards the n capacitors of the capacitor stack have to be charged again one after the other, which leads to discontinuous discharge of the capacitor outside the capacitor stack, which in turn is time consuming.

In various applications energy collection time can be crucial. A good example would be a clinical scenario of a pacemaker patient where there is a need to stimulate every single heartbeat over extended time periods of weeks, months and even years. In such a clinical scenario the harvesting electronics need to be fast enough to re-collect the energy needed for every stimulation within the time-period of every preceding cardiac cycle.

Besides electronics, the collection time is also a function of the tissue interface design. At the moment devices as described in WO2021/089530 A1 use microneedles as tissue interface. These microneedles are in contact with the tissue and are able to collect low charges from multiple cells in the tissue. The amount of charge which is collected depends on the design of these microneedles and is limited by them. The same design of microneedles is described in connection with a cardiac pacemaker WO 2021/089531 A1 and a neural tissue stimulator WO 2022/229370. Increasing the number of microneedles will increase the amount of charge collection and reduce the collection time needed. It, however, comes at the cost of increased device sizes - countering the efforts of device miniaturization.

Other state-of-the-art studies for collecting charges have used glass micropipette electrodes to access the electrical signal inside a biological source. This tissue interface approach led to high source impedances and limited energy availability [5-7]. Two studies were single-cell patch clamp experiments in an ex-vivo lab environment [6, 7]. They recorded -60 mV source signals and harvested them by directly connecting to a capacitor. Consequently, the maximum harvested voltage was equal to the membrane potential, relatively low, and would not be able to power integrated electronic circuits. The only other in-vivo study was done in 2012 [5]. It accessed endocochlear potentials in a guinea pig model, which are DC potentials of around 70 to 100 mV. An inductive boost converter was used to harvest the energy and transform the harvested voltage to levels high enough to power electronic circuits. Due to the high source impedances it was only possible to harvest energy of around 20 µJ over 300 minutes of harvesting time, which would be much too little power.

Therefore, it is the purpose of the invention to overcome the above-mentioned disadvantages of the state of the art and to provide a device which enables the tissue interface to collect as much charge as possible from multiple cells in a tissue.

Therefore, the invention provides a tissue interface according to claim 1, a cardiac pacemaker according to claim 10 and a method for stimulating myocardial tissue according to claim 12.

### Detailed description

### Tissue interface

The tissue interface according to the invention is adapted to contact with the electrochemical potentials inside a biological tissue which represents a voltage source inside a tissue.

Most preferably the electric potential of the voltage source are between 1 mV and 500 mV, more preferably between 2 mV and 100 mV, most preferably between 10 mV and 30 mV and are cardiac monophasic action potentials or other electrograms resulting from depolarization and repolarization periods of bioelectrical cell and tissue activity.

A voltage source inside a tissue are cell potentials from living cells. Bioelectric signals of tissue cells typically have an electric potential of 10 - 90 mV and an internal resistance of 0.1 to 200 kOhm, wherein most often bioelectric signals of tissue cells have an internal resistance of 500 to 5000 Ohm. For example, bioelectric signals of nerve cells have typically an electric potential of 60 mV and an internal resistance of 20 to 200 kOhm.

In one embodiment the tissue interface according to the invention uses cell potentials from living cells as voltage source. The tissue used for energy collection can be myocardial tissue, neural tissue, skeletal muscle tissue, smooth muscle tissue (e.g. inside the gastrointestinal tract), skin tissue, for example.

The tissue interface comprises at least one microneedle or one microcable.

In one embodiment the at least on microneedle or the at least one microcable comprises a material selected from stainless steel, Platin/Iridium (Ptlr), gold, fine metals, refractory metals, conductive organic materials and conductive plastics or is made from one of these materials. In a preferred embodiment the microneedle or microcable consist of stainless steel.

In one embodiment the tissue interface further comprises at least one microcable or microneedle which is adapted to function as reference electrode. The at least one microcable or at least one microcable functions as reference electrode due to the tissue compartment it contacts.

According to the invention a part of the at least one microneedle or the at least one microcable has an isolating layer. The isolating layer surrounds a part of the surface of the microneedle or microcable. In one embodiment the isolating layer comprises a material selected from the group comprising organic materials or is made from one of these materials. Suitable organic materials are for example plastics which are hardening through esterification or evaporation of solvents after applying. In one embodiment the isolating layer comprises a material selected from the group comprising polyurethane, parylene and silicone or is made from one of these materials. In one embodiment the isolating layer can be applied as lacquer or varnish.

The partial isolating layer minimizes direct coupling between neighboring microneedles or microcables. This is especially needed when neighboring interfaces are located in different electrical tissue compartments (e.g., myocardium versus blood pool). As an example, direct coupling can take place between non-isolated neighboring microneedles or microcables when the harvesting interface is placed inside the tissue source compartment (e.g., inside the myocardium) and the reference is placed in a different compartment (e.g., the blood pool). Direct coupling between harvesting and reference electrode leads to undesired current shunting outside the harvesting electronics and reduces the amount of collectable charge and harvestable energy per tissue interface. The partial isolation approach reduces direct coupling and subsequent current shunting.

According to the invention a part of the at least one microneedle or at least one microcable has a coating. The coating can be PVD (physical vapor deposition) or atomic layer deposition, or surface texturing to increase the surface volume of the at least one microneedle or at least one microcable. In one embodiment of the invention the coating is selected from the group comprising Titanium Nitride.

In a preferred embodiment the coating has a porous or fractal surface.

The transfer of the charge of a voltage source via the tissue interface can be further improved by increasing the Helmholtz double layer capacitance of the microneedles or microcables. Hence, the surface of microneedles or microcables matters, as it defines the specific Helmholtz double layer capacitance C'_{H}. The coating introduces a porous or fractal surface which increases the electrochemical surface area and thereby the specific Helmholtz double-layer capacitance. This capacitance can be increased from about 0.02 µF/mm² with smooth surface designs of the microneedle or microcable to C'_{H} values of about 40 µF/mm² with porous or fractal surface designs.

The part of the surface of the microneedles or microcables which is not isolated is defined as the exposed surface area, which is in direct contact with the excitable tissue. The specific Helmholtz double-layer capacitance C'_{H} multiplied with the exposed surface area of a microneedle or microcable defines the entire Helmholtz double-layer capacitance for an entire microneedle or microcable.

The partial isolating layer allows the exposed surface area of the microneedles or microcables to only contact their intended electrical target compartment. For example, in case the tissue interface according to the invention is used in a cardiac pacemaker one microneedle or microcable can function as reference electrode in the blood pool and therefore has to be isolated against the myocardium. Other microneedles or microcables can function as harvesting/sensing/pacing electrodes and therefore have to be confined within the myocardium and isolated against the blood.

With a larger non-isolated surface area of a harvesting microneedle or microcable more exposed electrode surface area is in contact with the electrical tissue source. Consequently, with a larger non-isolated surface area more charge can be collected from a microneedle or microcable.

In one embodiment in this way the exposed surface area can be in the range of 3 mm² to 50 mm² per microneedle or microcable, most preferably between 5 to 15 mm².

For optimal results the exposed surface area of a microneedle or microcable should be as large as possible, wherein the coupling between neighboring microneedles or microcables should be as minimal as possible.

In one embodiment the device according to the invention comprises at least one microneedle. Every microneedle has a proximal and a distal end. In one embodiment of the invention, the microneedle according to the invention has a proximal end, which is shaped cylindrical with a diameter between 0.05 mm and 0.5 mm, preferably the proximal end has a diameter of 0.1 mm and a height between 0.05 mm and 0.5 mm.

In a further embodiment of the invention the microneedle according to the invention has a proximal end which is shaped like a cuboid with a width and depth between 0.05 mm and 0.5 mm, preferably the width and depth of the cuboid is 0.1 mm. The height of the cuboid is between 0.05 mm and 0.5 mm, preferably the cuboid has a height of 0.2 mm.

From the proximal end, the needle comprises a tapered portion which connects the distal end with the proximal end. The distal end is needle shaped and has a length between 0.5 mm and 10.0 mm. The distal end of the needle is electrically conductive and shear stress resistant in the range of 5 to 50 Newton, which is comparable to the shear stress resistance of bonding wires. In one embodiment the microneedle is milled from one piece and afterward the isolating layer and the coating are applied.

Preferably, the diameters of the distal ends of the microneedles are between 0,001 mm and 0.1 mm, preferably between 0.01 mm and 0.1 mm, most preferably the diameters of the distal ends of the microneedles are 0.02 mm. Thereby, the distal ends of the microneedles approximate cellular dimensions. The dimensions of the microneedles are therefore a lot smaller than any other electrodes in use today.

In one embodiment the device according to the invention comprises at least one microcable. The microcables are also called filaments. Each microcable comprises a wire, having a width in the range from 20 to 400 µm and a length in the range from 0.5 to 15 cm.

If the microcable is too floppy to be inserted into the tissue, in one embodiment an assistance device can be used to insert the microcable. The assistance device comprises a stiffer carrier structure and is either removed after deployment of the microcable or dissolves after deployment of the microcable. In one embodiment the microcable comprises a material which is stiff enough to insert the microcable into the tissue or is made of such a material. In a further embodiment the microcable comprises a material with a memory function or is made of such a material. In this embodiment the microcable takes a certain shape after deployment in the tissue. A suitable material is nitinol, for example.

Preferably, the microcables comprise conductive materials such as metals, conductive organic materials and conductive plastics. Suitable conductive organic materials are for example graphene and suitable conductive plastic materials are for example soot particles. In one embodiment parts of the microcables are coated and have therefore a fully or partially surrounding isolation layer to isolate portions of the interface against each other to avoid current shunting as already described above. Therefore, the wires are surrounded by a cover. The cover can be applied as lacquer or varnish. Therefore, the cover can comprise organic materials e.g. plastics which are hardening through esterification or evaporation of solvents after applying.

In one embodiment a part or the whole of the at least one microneedle or the at least one microcable has the structure of a helix or a 3D ball or 3D cage.

If the microneedles or microcable have the structure of a helix the electrode surface area in a certain 3D volume of tissue is increased through the turns of the helix. In one embodiment the helix has one turn which is beneficial for tissue which is thin and has not much space to accommodate a longer helix. In one embodiment only one of the microneedles or only one of the microcables of the tissue interface has the structure of a helix.

In a further embodiment the at least one microneedle or at least one microcable has the structure of a 3D ball or 3D cage which maximizes 3D space utilization for electrode contact inside a box of tissue.

Thus the exposed surface area of the microneedle or microcable usable for harvesting is increased as well leading to higher amounts of charge collection. This further improves the harvesting efficiency of the inventive tissue interface.

In one preferred embodiment the tissue interface comprises an array of 1 to 10000, preferably 2 to 1000, most preferably 10 to 250 microneedles or microcables. In this embodiment the microneedles or microcables can form a bed of microneedles or microcables.

In one embodiment the length of at least one microneedle or microcable differs from the length of the other microneedles or microcables. In this embodiment individual microneedles or microcables can be inserted into the tissue up to a defined area. Accordingly, not alle microneedles or microcables penetrate the tissue equally deep.

Preferably, all materials comprised in the microneedles or microcables are bio-compatible and insensitive to body liquids. Bio-compatible in conjunction with the present invention means that no toxic interactions occur between the bio-compatible material and tissue, e.g. human tissue.

The following features are optimized for the design of the microneedles or microcables inventive tissue interface:
(i) microneedle core material,
(ii) undesired direct coupling between different microneedles or microcables (in case of using the tissue interface as part of a cardiac pacemaker undesired coupling between harvesting-to-harvesting and harvesting-to-reference electrodes is prevented),
(iii) specific Helmholtz double layer capacitance, and
(iv) exposed surface area of the microneedle or microcable to a tissue for harvesting.

### Interposer layer

In one embodiment the tissue interface comprises an interposer layer comprising a hole for each microneedle or microcable.

In one embodiment of the invention the interposer layer comprises holes, each hole being suitable for the distal end of a microneedle or microcable to pass through. In one embodiment the interposer layer comprises a hole for each microneedle or microcable of an array of microneedles. Thus, in a preferred embodiment the interposer layer comprises as many holes as an array of microneedles or microcables comprises microneedles or microcables.

In a further embodiment of the invention the proximal end of each microneedle or microcable is soldered to the surface of the interposer layer. Further, the distal end of every microneedle or microcable protrudes the interposer layer.

The interposer layer comprises a material of the group comprising FR4 materials, epoxy-resin, Poly(methyl methacrylate) (PMMA), ceramics, silicon dioxide (SiO₂), glass and plastics. FR4 materials are a class of flame-resistant composite materials comprising woven fiberglass and epoxy resin. Principally, the materials comprised in the interposer layer have to be non-electrically conductive. Further, according to the invention all materials comprised in the interposer layer are bio-compatible and insensitive to body liquids.

Every hole in the interposer layer with a microneedle or microcable passing through is sealed to the surrounding with a non-conductive material. Suitable non-conductive materials are for example epoxy-resin, Poly(methyl methacrylate) (PMMA), glass and plastics. Thereby, no fluids from the environment can penetrate through the holes.

### Cardiac pacemaker

All features described above apply for the cardiac pacemaker as well and vice versa.

The invention further provides a cardiac pacemaker, characterized in that the cardiac pacemaker comprises
a tissue interface according to the invention comprising an array of microneedles or microcables;
at least one reference electrode;
a chip comprising at least one comparator with adaptive level, a sequence control circuit, at least one capacitor stack built by *n* capacitors and 2n switches, at least one buffer capacitor outside the at least one capacitor stack, at least two additional switches outside the at least one capacitor stack and a CMOS-Logic, wherein *n* ∈ *N* and *n* ≥ 1;
an interposer layer comprising holes for the microneedles or microcables of the tissue interface;
a lid;
at least one coil;
wherein the chip, is located on one surface of the interposer layer;
wherein the lid and the interposer layer form a capsule for the chip and the at least one coil;
wherein each microneedle or microcable has a distal end which protrudes from the chip; and
wherein the cardiac pacemaker is adapted to be electrically self-sufficient due to harvesting of electrical energy from myocardial cells.

The following applies in principle, *n* ∈ *N* and *n* ≥ 1 with *N* being positive integers *N =* {1,2,3, ...}.

The cardiac pacemaker according to the invention comprises a tissue interface according to the invention comprising an array of microneedles or microcables.

The small dimensions of the microneedles or microcables of the tissue interface offer several advantages over the state of the art. Firstly, microneedles or microcables according to the invention couple directly electrically to the extracellular ionic fluid space around a limited number of heart muscle cells. Thereby, one microneedle or microcable is in close electrical contact with the ionic fluid environment around about 100 heart muscle cells. Hence, the electrical signals of the microneedles or microcables according to the invention are near-field signals and are similar in form to the action potential of individual heart muscle cells. In result, microneedles or microcables according to the invention are able to sense surrogates of cellular electrical activity (so called monophasic action potentials), harvest energy directly from inside the myocardial tissue and/or emit electrical pulses directly into the myocardial tissue due to their small dimensions. Thereby, advantageously the stimulation threshold is lowered and the medical signal interpretation is enhanced through insights into cardiac depolarization and repolarization. State of the art devices are not suitable for this purpose. Their electrodes produce far-field signals from traveling wavefronts because they are 10 to 30 times larger, float above the tissue or are encapsulated, and they lack direct ionic access to the extracellular intramyocardial space. In one embodiment the length of at least one microneedle or microcable differs from the length of the other microneedles or microcables. In this embodiment individual microneedles or microcables can be inserted into the tissue up to a defined area. Accordingly, not all microneedles or microcables penetrate the tissue equally deep.

The isolating layer allowed the exposed surface area of the microneedles or microcables to only contact the desired tissue in their intended electrical target compartment. In one embodiment at least one microneedle or microcable functions as reference electrode in the pacemaker and is therefore isolated against the epicardium, endocardium, and myocardium, while all other microneedles or microcables function as sensing/pacing and/or harvesting electrodes and are therefore not isolated within the myocardium.

In a preferred embodiment the length and diameter of the microneedles or microcables were balanced to
(i) support compartmentalized location of the microneedles or microcables;
(ii) maximize the surface area of the microneedles or microcables inside a desired compartment;
(iii) reduce tissue defense process.

The cardiac pacemaker further comprises at least one reference electrode. In one embodiment the reference electrode is a microneedle, a microcable, a ring, a bump, an eyelet or a button, . In one embodiment the reference electrode is part of the blood exposed surface of the device. If the reference electrode is a microneedle or microcable all features already described for the microneedles and microcables of the tissue interface apply for the reference electrode as well.

The reference electrode works as such due to the tissue compartment it contacts. In a preferred embodiment the reference electrode contacts the intracardiac blood pool.

In one embodiment the reference electrode is comprised in the tissue interface.

In a further embodiment the reference electrode is arranged on a side of the cardiac pacemaker, which does not comprise the tissue interface. In a preferred embodiment the reference electrode is arranged on the side of the cardiac pacemaker that is opposite the side comprising the tissue interface.

In a preferred embodiment of the invention the tissue interface of the cardiac pacemaker comprises between 1 and 10000 microneedles or microcables, preferably between 2 and 1000 microneedles or microcables, most preferably between 10 and 250 microneedles or microcables.

Principally the microneedles or microcables can be arranged on the chip in every way. In a preferred embodiment of the invention, the microneedles or microcables are arranged symmetrically to each other on the chip. Thereby, advantageously the largest possible number of microneedles or microcables can be arranged on the surface of the chip. Further, the regularity in the order of the microneedles or microcables simplifies production processes.

Further, preferably, each microneedle or microcable is adapted to be able to harvest cellular energy, to electrically stimulate live tissue and to sense intrinsic cellular electrical activity. According to the invention, every microneedle or microcable is operable independent of the other microneedles or microcables. Which means that one microneedle or microcable could harvest energy while a neighboring microneedle or microcable is sensing intrinsic cellular electrical activity. The tasks of each microneedle or microcable can be redistributed at any time and thus adapted to the current requirements of the cardiac pacemaker.

Further, the cardiac pacemaker according to the invention comprises a chip and an interposer layer. The features described above for the interposer layer apply for the cardiac pacemaker as well. In one embodiment of the invention the proximal end of each microneedle or microcable is soldered to the surface of the chip, which ensures that each microneedle or microcable has a direct contact to the chip. In a further embodiment of the invention the proximal end of each microneedle or microcable is soldered to the surface of the interposer layer of the cardiac pacemaker. Further, the distal end of every microneedle or microcable protrudes from the chip and/or the interposer layer.

According to the invention the chip comprises all devices necessary to control the cardiac pacemaker's functions. Therefore, the chip comprises at least one comparator with adaptive level, a sequence control circuit, at least one capacitor stack built by *n* capacitors and 2*n* switches, at least one buffer capacitor outside the at least one capacitor stack, at least two additional switches outside the at least one capacitor stack and a CMOS-Logic, wherein *n* ∈ *N* and *n* ≥ 1 with *N* being positive integers *N* = {1,2,3, ... }.

### Self-sufficiency energy harvesting

According to the invention the 2*n* switches of the at least one capacitor stack couple the *n* capacitors selectively to at least one microneedle or microcable and thereby to the voltage source. Further, the *n* capacitors of the at least one capacitor stack are dedicated to be sequentially charged by at least one microneedle or microcable one after the other. And the at least one buffer capacitor outside the at least one capacitor stack is dedicated to be charged from the *n* capacitors of the capacitor stack at once.

Hence, the chip according to the invention comprises at least one capacitor stack, wherein the capacitor stack is built by *n* capacitors and 2*n* switches, wherein *n* ∈ *N* and *n* ≥ 1 with *N* being positive integers *N* = {1,2,3, ... }.The capacitor stack can comprise as much capacitors as can be accommodated constructively. In one embodiment of the invention k is between 2 and 20, more preferably between 2 and 14. The *n* capacitors of the capacitors stack are dedicated to be sequentially charged via at least one microneedle or microcable by the voltage source, one after the other.

The 2*n* switches of the capacitor stack couple the *n* capacitors selectively to at least one microneedle or microcable in a way that every capacitor is sequentially charged by the voltage source made available by the at least one microneedle or microcable one after the other. The voltage input is made available since the microneedles or microcables couple directly electrically to heart muscle cells and derive the electrical signal. The controlling and sequencing of the switches is generated from a usual CMOS-Logic, which is common to Microelectronics.

At least one buffer capacitor is situated outside the capacitor stack, which works as a buffer. According to the invention, the at least one buffer capacitor is dedicated to be charged from the n capacitors of the at least one capacitor stack at once. In a preferred embodiment of the invention, the chip comprises one buffer capacitor outside the capacitor stack. In a further preferred embodiment of the invention the chip comprises two buffer capacitors outside the capacitor stack.

Furthermore, the chip comprises at least two additional switches outside the capacitor stack. In a preferred embodiment of the invention the chip comprises two additional switches outside the capacitor stack. The additional switches are dedicated to selectively couple the capacitor stack to the at least one buffer capacitor outside the capacitor stack or to a further optional capacitor stack.

In a further preferred embodiment the chip comprises four additional switches outside the capacitor stack. Preferably the chip comprises four additional switches outside the at least one capacitor stack if the chip comprises a first buffer capacitor outside the at least one capacitor stack and a second buffer capacitor outside the at least one capacitor stack. In this embodiment two additional switches are dedicated to selectively connect the at least one capacitor stack to the first buffer capacitor outside the capacitor stack and the two further additional switches are dedicated to selectively connect the at least one capacitor stack to the second buffer capacitor outside the capacitor stack.

Accordingly, in one preferred embodiment the chip according to the invention comprises two buffer capacitors outside the capacitor stack as buffer capacitors outside the at least one capacitor stack and four additional switch outside the at least one capacitor stack.

From its physical construction as a stack, the n capacitors of the capacitor stack are all connected in series electrically. Furthermore, in one embodiment of the invention, the at least one capacitor stack comprises at least three conductive plates wherein the conductive plates have a top-side and a bottom-side and wherein the top-side of at least one conductive plate is part of a first capacitor and the bottom-side of the at least one conductive plate is part of a neighboring further capacitor. Furthermore, the capacitor stack comprises an isolating material between the conductive plates in a way that a capacitor is built.

In a preferred embodiment of the invention, a capacitor stack with n capacitors comprises m=n+1 conductive plates. According to the invention the first conductor n=1 is built between the bottom-side of the first conductive plate (m=1) and the top-side of the second conductive plate (m=2). The neighboring conductor (n=2) is built between the bottom-side of the second conductive plate (m=2) and the top-side of the third conductive plate (m=3) and so on.

The capacitance of the capacitors built according to the invention is quite wide ranging from 1 nF down to 1fF and even below. It depends on plate geometries and the dielectric material employed between the plates. Typical dielectric materials are SiO2 or plastic, but other dielectric materials are possible.

The arrangement of the conductors in a capacitor stack with n capacitors according to the invention has the advantage that the inner conductive plates, which means plates m=2 to m=n form no or just very small parasitic capacitances to the outside of the stack. Parasitic capacitances are well known in the art. They arise at the interfaces of capacitors to the surrounding and are unwanted as those have to be charged at every charge cycle of the capacitor. This process lowers the charging efficiency of the capacitor and therefore its end-charging voltage. Accordingly, in the state of the art every capacitor has two interfaces to the surrounding and therefore two interfaces where parasitic capacitances arise.

The capacitor stack according to the invention is able to provide n capacitors, wherein only the first and the last capacitor have a substantial interface to the surrounding. Therefore, advantageously, only at these two interfaces parasitic capacitances will form. Accordingly, the charging efficiency of the n capacitors of the capacitor stack is increased as well as the end-charging voltage.

Furthermore, in a preferred embodiment of the invention, all capacitors are connected in series electrically.

In one preferred embodiment of the invention the at least one capacitor stack built by n capacitors and 2n switches, the at least one buffer capacitor outside the at least one capacitor stack and the at least two additional switches outside the at least one capacitor stack are configured as an integrated circuit wherein switches are realized as transistors and capacitors are realized by conductive plates from integrated circuit technology.

Preferably the conductive plates are made of material selected from the group comprising metal or polysilicon or any other conductive material from integrated circuit technology. Suitable metals are copper and aluminum and tungsten.

In one embodiment of the invention the isolating material is selected from the group comprising SiO₂, SiN and Hf₂O and stacks thereof.

As described above the capacitor stack is internally nearly perfect if it comes to storing the applied charges, as the field is nicely confined internally. Unfortunately at the first and last conductive plates still some parasitic capacitances will form. In view not to lose the energy stored in those external parasitic capacitances, according to the invention, an inductor can be applied to perform intermediate storage in a resonant circuit configuration.

In another preferred embodiment the chip comprises several capacitor stacks wherein every capacitor stack is dedicated to charge another capacitor stack and one capacitor stack is dedicated to charge at least one buffer capacitor outsides the capacitor stacks. Thereby, cascading of the sequential small charge collection according to the invention is possible.

In an embodiment of the invention the sequencing of the switches is generated from a usual CMOS-Logic, which is common to Microelectronics. For the CMOS-logic to function, voltages of a few hundred millivolts are required. Typical state of the art semiconductor technology operates at around 1 Volt or slightly below. Since the cardiac pacemaker according to the invention collects energy starting with a few millivolts at the source, this voltage is too low to operate the CMOS-logic.

### Startup circuit

However, after collection and cascading, voltages in the 1-Volt domain can be obtained, which is enough to operate the CMOS-logic. For this reason, a startup circuit is required, to make sure the logic can be powered and the switches are operated to perform energy collection from the tiny sources.

For this, a magnetic coupling over coils is proposed. The outer coil is excited with alternate current, creating a magnetic alternating field. Through this field the startup energy is transmitted to the coil on the integrated circuit, which recuperates the startup energy.

According to the invention the cardiac pacemaker comprises at least one coil. Preferably the coil is dedicated to receive a startup energy by magnetic coupling with another coil. In one embodiment the at least one coil is located on the interposer layer. In a further embodiment of the invention the at least one coil is part of the chip in the sense that the at least one coil is wound around the chip. However, in each of these embodiments the chip comprises an interface for power management to connect the at least one coil of the cardiac pacemaker to the CMOS-logic comprised on the chip.

In one embodiment of the invention the cardiac pacemaker comprises at least one further capacitor. The at least one further capacitor can be comprised on the chip or can be located on the interposer layer outside the chip. The at least one further capacitor of the cardiac pacemaker serves preferably as buffer capacitor for all microneedles of the array of microneedles and the capacitor stacks assigned to them. Furthermore, the at least one further capacitor of the cardiac pacemaker can serve for energy transfer from an external energy source, e.g. for the startup process. In one embodiment the at least one further capacitor is connected to the chip by the interface for power management comprised on the chip.

### Programmability

Further, the chip of the cardiac pacemaker according to the invention is adapted to communicate with an external programmer unit. External means that the unit must not be in direct contact with the patient at all. The communication is preferably done via externally applied electromagnetic fields. Accordingly, in one embodiment the cardiac pacemaker further comprises at least one coil to communicate with the external programmer unit, thereby the coil functions as receiver and transmitter. Furthermore, the chip comprises an I/O interface for data transmission from the external programmer unit via the coil of the cardiac pacemaker to the chip. This has the advantage, that the functionality of the cardiac pacemaker can be proofed, surveyed and adjusted from the external programmer unit. Hence, adjustments in the functionality of the cardiac pacemaker are possible through the tissue and without physical contact to the cardiac pacemaker. Accordingly in a preferred embodiment of the invention the cardiac pacemaker further comprises an external programmer unit. The external programmer unit is selected from a group comprising tablets, smartphones and PC's. The external programmer unit is adapted to communicate with the cardiac pacemaker, therefore in a preferred embodiment a coil for transmitting and receiving is comprised in the external programmer unit.

Accordingly in one embodiment of the invention the cardiac pacemaker comprises at least two coils, wherein one coil is adapted to receive a startup energy for the CMOS-logic and one coil is adapted to function as receiver and transmitter to the external programmer unit. Both coils can be located on the interposer layer or can wound around the chip as already described. In another embodiment one coil can be located on the interposer layer and another coil can be wound around the chip.

Advantageously, in a preferred embodiment the coil which is comprised in the cardiac pacemaker and uses to communicate with the external programmer unit and the coil which is comprised in the cardiac pacemaker and used to receive a startup energy for the CMOS-Logic are the same coil.

### Interposer layer

Further the cardiac pacemaker according to the invention comprises an interposer layer and a lid. All features of the interposer layer described above also apply for the cardiac pacemaker and vice versa.

The chip is positioned on top of the interposer layer and the microneedles or microcables pass through the holes in the interposer layer. Hence, every microneedle or microcable passes through a separate hole in the interposer layer. Advantageously, the holes in the interposer layer are arranged in a way that all microneedles or microcables which are soldered to the chip can pass through without making contact to the interposer layer.

In a further embodiment each microneedle or microcable is soldered to the interposer layer. In this embodiment the interposer layer comprises a wiring connecting each microneedle or microcable to the chip, thereby connecting each microneedle or microcable to at least one capacitor stack.

In one embodiment the cardiac pacemaker further comprises at least one further capacitor and/or at least one sensor. In this embodiment the interposer layer comprises a wiring connecting the chip, the at least one senor and the at least one further capacitor with each other.

Further, in one embodiment the interposer layer serves as assembly platform for the at least one further capacitor and/or the at least one sensor and/or the at least one coil of the cardiac pacemaker. In this embodiment the at least one further capacitor and/or the at least one sensor and/or the at least one coil are preferably positioned next to the chip on the interposer layer.

According to the invention the cardiac pacemaker further comprises a lid. The lid covers the chip from the surrounding, wherein the lid is sealed to the interposer layer. Sealing can be done by adhesives or soldering tin. If adhesives are used the adhesive should be hardened. However, the sealing should be bio-compatible and insensitive to body fluids. Accordingly, the lid and the interposer layer form a capsule for the chip. The lid and the interposer layer shield the electronic parts from surrounding body-fluids like blood, e.g. from body fluids by forming a capsule.

In one embodiment of the invention next to the chip on top of the interposer layer at least one sensor and/or at least one capacitor and/or at least one coil are positioned. The chip, the at least one sensor and/or the at least one capacitor and/or the at least one coil are located on one surface of the interposer layer and the lid covers the chip, the at least one sensor and/or the at least one capacitor and/or the at least one coil from the surrounding, wherein the lid is sealed to the interposer layer, as already described. Thereby, the lid and the interposer layer form a capsule for the chip, the at least one sensor and/or the at least one capacitor and/or the at least one coil. Preferably, the lid and the interposer layer shield all electronic parts comprised in the cardiac pacemaker from surrounding body-fluids like blood, e.g. from body fluids by forming a capsule.

The lid comprises a material of the group comprising silicon and metals. Suitable metals are for example aluminum, or aluminum vaporised with tungsten. According to the invention all materials comprised in the lid are bio-compatible and insensitive to body liquids.

The interposer layer further comprises at least two fixing holes outside the lid. Advantageously, a fixing hole is positioned outside the lid on each side of the lid in the interposer layer. The fixing holes are suited to serve for the fixation of the cardiac pacemaker into myocardial tissue. The cardiac pacemaker can be fixated by screws, clamps or such like devices through the fixing holes in the interposer layer outside the lid.

In one embodiment of the invention the cardiac pacemaker further comprises at least one sensor. In a preferred embodiment of the invention the sensor is an activity sensor. The activity sensor may include an accelerometer, such as an electrostatical accelerometer, a piezoceramic accelerometer or a MEMS-based micromechanical accelerometer, that typically provides a sensor output that varies as a function of a measured parameter relating to a patient's metabolic requirements. In other words, activity sensors detect motion of the patient that accompanies physical activity and may adjust the pacing rate to the metabolic needs associated with the physical activity. In addition, the activity sensor may be configured to detect a change in the posture of a patient. If the cardiac pacemaker comprises at least one sensor the chip of the cardiac pacemaker comprises a sensor interface to connect the sensor with the chip.

In a preferred embodiment of the invention the cardiac pacemaker comprises a MEMS-based three-vector activity sensor.

The cardiac pacemaker according to the invention is between 1 mm and 5 cm long, between 1 mm and 5 mm wide and between 1 mm and 10 mm high. Thereby, the cardiac pacemaker according to the invention is smaller than any currently available cardiac pacemaker. This offers several advantages, first of all a cardiac pacemaker of this size can be implanted more easily as it allows easier access via the vein. Due to its small dimensions the cardiac pacemaker according to the invention grows better into the tissue, is less susceptible to infections and is less prone to heart perforations.

In one embodiment the cardiac pacemaker has the shape of a cylinder. The cardiac pacemaker has a diameter between 1 and 10 mm, preferably between 2 and 4 mm, most preferably between 2 and 3 mm.

Advantageously, the cardiac pacemaker according to the invention can be positioned via transcatheter into the heart chamber. Accordingly, the tissue interface contacts the tissue of the heart wall from the side of the endocardium. The reference electrode contacts the intracardiac blood pool and is not in contact with the heart wall. Due to the delivery by transcatheter the surgical procedure is simplified and post-surgery complications are reduced. A shorter, less invasive implant procedure can be provided which improves the therapy as a whole.

According to the invention the tissue interface is preferably able to sustainably sense and record specific intramyocardial electrograms so called monophasic action potentials.

Advantageously the cardiac pacemaker provides information of depolarization and repolarization within the cardiac activation cycle.

Since the cardiac pacemaker according to the invention is adapted to sense cellular electrical activity and to generate an electrical pulse if necessary, the cardiac pacemaker according to the invention provides all functions that are required by a cardiac pacemaker. Advantageously, the cardiac pacemaker according to the invention is adapted to be electrically self-sufficient.

According to the invention, the chip comprises a sequence control circuit. This circuit controls the functionality of the cardiac pacemaker and determines the workflow of the functions of the cardiac pacemaker. All interfaces comprised on the chip like I/O-interface, sensor interface and interface for power management are interfaces to the sequence control circuit.

### Sensing

Sensing of cellular electrical activity requires measurement of the amplitude of the actual potential with regards to the reference electrode. Generally, this is performed with a comparator circuit. For cardiac pacemakers it is important to know the timing of the expected/intended heartbeat. The measurement of the myocardial potential is standard and in use with current cardiac pacemakers.

According to the invention, the chip comprises at least one comparator with adaptive reference level. In a preferred embodiment of the invention the chip comprises a comparator with adaptive reference level for each microneedle or microcable of the tissue interface, wherein each microneedle or microcable is electrically connected to one comparator circuit on the chip.

According to the invention, every microneedle or microcable is adapted to be able to sense the amplitude of the intrinsic cellular electrical activity by the standard procedure of measurement of myocardial potential. Advantageously, these measurements can be performed on one microneedle or microcable, on selected microneedles or microcables or on all of the microneedles or microcables of the tissue interface. The redundancy of microneedles and microcables in the tissue interface provides a number of beneficial features. How many and which of the microneedles of the array of microneedles perform sensing is programmed via the external programmer unit.

In one embodiment the cellular electrical activity is sensed continuously. In a further embodiment of the invention the cellular electrical activity is sensed in a way that it is monitored when the amplitude of the cellular electrical activity exceeds the reference level, when the amplitude of the cellular electrical activity falls below the reference level and what the maximum amplitude of the cellular electrical activity during a heart cycle is. Thereby, timing points of the individual progression of the heartbeat are monitored. Further, the amplitude of the cellular electrical activity is sensed by at least one or microcable of the tissue interface and recorded by the external programmer unit or in one embodiment by an internal data memory device, which is comprised in the cardiac pacemaker. In case the amplitude of the cellular electrical activity is recorded by an internal data memory device, the chip according to the invention further comprises a suited internal memory device.

Further, the amplitude of the actual myocardial potential sensed by the microneedles or microcables of the tissue interface selected for sensing of cellular electrical activity is compared to a reference level by the at least one comparator. Principally the reference level for each comparator is programmable. In one embodiment of the invention a reference level is programmed individually for each microneedle or microcable of the tissue interface. In a further embodiment of the invention, the reference level is the same for each comparator comprised on the chip. In one embodiment of the invention the reference level of the at least one comparator is programmable between 0.1 mV and 10.0 mV.

Hence, the reference level can be the same for all microneedles or microcables sensing the cellular electrical activity but can also be different for each microneedle or microcable. By programming the reference level of each comparator individually, optimized timing points for all microneedles or microcables of the tissue interface which are penetrating the tissue at different positions can be provided. This is very advantageous, since the timing of all functions of the cardiac pacemaker according to the invention can be programmed according to individual progression of the heartbeat. This increases on the one hand safety aspects, since the cellular electrical activity is sensed by several individual microneedles or microcables, which can be understood as sensing with several individual electrodes. If one microneedle or microcable does not function correctly another microneedle or microcable can be programmed to perform sensing function. Accordingly, sensing function is provided in a redundant way. In contrast, in state-of-the-art cardiac pacemakers only one electrode is provided for sensing cellular electrical activity. If this electrode does not function correctly, the pacemaker has to be replaced. Hence, the cardiac pacemaker according to the invention reduces the risk of new operations.

On the other hand, in one embodiment of the invention the amplitudes of the cellular electrical activity sensed by several microneedles or microcables of the tissue interface are compared by the external programmer unit and/or by the sequence control circuit. Hence, electronic malfunctions/misinterpretations of the heart's own signal become less likely because permanently the signals of several microneedles or microcables are compared electronically.

Further, the efficiency of the cardiac pacemaker is increased compared to state-of-the-art cardiac pacemakers, since the microneedles or microcables used for performing energy harvesting and/or sensing of cellular electrical energy and/or for emitting an electrical pulse can be selected depending on faultless function of the respective microneedles or microcables of the tissue interface.

Due to the multiple microneedles or microcables sensing the cellular electrical energy independent from each other and which sense temporally and spatially aligned, the typical vulnerability of state-of-the-art cardiac pacemakers to electrical signal misinterpretation due to external electrical noise or electrical far-field signals is reduced.

Furthermore, improving the tissue interface technology according to the invention further enhances electrical signal recording capabilities from living tissue. Besides harvestable energy amounts those electrical signal recordings contain relevant clinical information on the status and well-being of the target tissue/organ. This information is useful to monitor normal biologic behavior, detect abnormalities, trigger medical therapies and surveille clinical follow-up. It can be meaningfully combined with therapeutic pacing capabilities. The monophasic action potential recorded from within the cardiac tissue and used for harvesting purposes is a classic example. Such electrogram recordings go beyond standard intra- and extracardiac ECG tracings. They are known to contain further information on resting electrical tissue potentials, as well as depolarization and repolarization of myocardial tissue. Known examples of clinical relevance include inherited repolarization disorders such as the Long-QT-Syndrom, repolarization abnormalities during myocardial ischemia and repolarization abnormalities predicting sudden cardiac death. Despite this knowledge current tissue interface technologies used in clinical device technologies have not been able sustainably record such dedicated electrical signals from an intimate electrical connection within the target tissue, e.g. from inside the myocardium. Accordingly, in one embodiment the cardiac pacemaker is configured to record and store bioelectric signals. The bioelectrical signals can be stored in a suitable memory inside or outside the cardiac pacemaker.

### Harvesting

According to the invention, every microneedle or microcable of the tissue interface of the cardiac pacemaker is adapted to harvest cellular energy. The heart is a big muscle and transforms chemical energy (nutrition) into mechanical energy (heartbeats). This process is controlled and conducted through the spread of electrical energy over all myocardial cells. The left main chamber of the heart (left ventricle) contains a total of approximately 6 billion cells. Each cell acts as a battery which is discharged and charged once during each cardiac cycle. That function is mediated by the exchange of Sodium and Potassium through ion channels in the cellular membrane. The actual electrical energy turnover of an individual cell is small, however, harvesting from multiple cells and multiple times can collect a significant amount of electrical energy.

Even if the cellular transmembrane voltages cannot be directly accessed, as the microneedles or microcables are too large to reach an individual intracellular space, portions of the produced cellular electrical energy are collectible from the outside adjacent extracellular space. As one microneedle or microcable touches a sequence of cells (approximately 100 cells) which operate synchronously, the collectable energy increases.

Harvesting is done by a method comprising at least one capacitor stack build by *n* capacitors and 2n switches, at least one buffer capacitor outside the capacitor stack, at least two additional switches and at least one microneedle or microcable in contact with a DC source, comprising the steps
a. the n capacitors of the capacitor stack are sequentially charged by coupling one capacitor after the other to at least one microneedle or microcable of the tissue interface by selectively closing the switches;
b. discharging the n capacitors of the capacitor stack into the at least one buffer capacitor outside the capacitor stack;
wherein the at least one microneedle or microcable couples directly electrically to heart muscle cell which act as voltage source. Steps a. and b. define one harvesting cycle.

The features, advantageous embodiments and methods of energy harvesting described above also apply to the cardiac pacemaker according to the invention.

In one embodiment of the invention the cardiac pacemaker is adapted to perform several harvesting cycles during a singular heartbeat in order to additionally optimize the harvested energy amount.

According to the invention energy harvesting can be done on one microneedle microcable, on several microneedles or microcables or on all of the microneedles or microcables of the tissue interface simultaneously.

Furthermore, in one embodiment of the invention a selection mechanism is implemented to select high yielding microneedles or microcables of the array of microneedles or microcables for energy harvesting and to discard low yielding microneedles or microcables. In one embodiment of the invention, it is programmed which microneedles or microcables of the tissue interface are utilized for energy harvesting. This is done via the external programmer unit. In a further embodiment of the invention the microneedles or microcables which are utilized for energy harvesting are selected due to the cellular electrical energy sensed by the individual microneedles or microcables. This is regulated internally by the sequence control circuit on the chip, advantageously no regulation by the external programmer unit is necessary. Thereby, the microneedles or microcables are selected for energy harvesting that achieve the highest energy yield.

In one embodiment the cardiac pacemaker further comprises a rechargeable battery.

According to the invention the cardiac pacemaker is adapted to be electrically self-sufficient due to harvesting of electrical energy from myocardial cells. The harvested energy is used to operate all functions of the cardiac pacemaker and/or to recharge a battery inside the cardiac pacemaker. Therefore the cardiac pacemaker of the present invention is electrically self-sufficient, which means electrically autonomous. All disadvantages associated with battery operation are eliminated. Hence, there is no need for any kind of external recharge procedure for a battery or even a whole replacement of a cardiac pacemaker by a new one due to an empty battery.

According to the invention, sensing of the amplitude of the cellular electrical activity and energy harvesting happen together on a microneedle or microcable. If a cellular electrical activity above the reference level is detected, harvesting of electrical energy starts. In one embodiment of the invention, harvesting is carried out with repeated charging cycles until the cellular electrical activity falls below the reference level. Which means the microneedle or microcable harvests cellular electrical energy multiple times throughout a singular cardiac cycle. In this embodiment the time interval for energy harvesting depends on the heart rate and is approximately between 150 ms and 300 ms. In a further embodiment of the invention harvesting is carried out until a programmable time-out is reached. In this case the programmable time-out could be between 200 ms and 300 ms.

In one embodiment the cardiac pacemaker is adapted to harvest energy by at least one microneedle or microcable of the tissue interface immediately after emitting a monophasic pulse. This is arranged by programming with the external programmer unit.

### Pacing

According to the invention, every microneedle or microcable is adapted to be able to emit an electrical pulse. The electrical pulse is suited to electrically stimulate live tissue. According to the invention, every microneedle or microcable of the tissue interface is able to emit a monophasic pulse in the tissue. The voltages of the electrical pulse typically range from 100 mV to 2 V and the pulse lengths varies from 0.2 ms to 2.0 ms. According to the invention the electrical pulse is generated utilizing the harvested energy. Emitting of an electrical pulse is controlled by the sequence control circuit on the chip.

After an electrical pulse is applied, a new heartbeat is initiated. Starting from the location in the heart, where the pulse is applied through the microneedle or microcable, the electrical activation and subsequent contraction propagates through the entire heart autonomously. No further pulse or action is required, once the pulse voltage exceeds the energy needed to activate the tissue.

As already described, in principle each microneedle or microcable is able to emit an electrical pulse. In a preferred embodiment of the invention one microneedle or microcable of the tissue interface is selected to emit an electrical pulse if necessary. The respective microneedle or microcable is selected via the external programmer unit.

In one embodiment of the invention, the functionality to emit an electrical pulse is programmable for each microneedle or microcable of the tissue interface. An algorithm-based comparison of stimulation thresholds selects the microneedle or microcable of the tissue interface with the lowest stimulation threshold. Advantageously, by utilizing the microneedle or microcable of the tissue interface having the lowest stimulation threshold minimizes the voltage of the electrical pulse emitted, which minimizes the energy consumption of the cardiac pacemaker.

While every microneedle or microcable is able to harvest cellular energy, to electrically stimulate live tissue and to sense intrinsic cellular electrical activity, the microneedles or microcables are not programmed to perform all three functionalities at the same time. Sensing of cellular electrical activity and harvesting of cellular energy is performed at one microneedle or microcable at the same time as already described. In one embodiment of the invention, sensing of cellular electrical energy and emitting an electrical pulse is done at the same microneedle or microcable of the tissue interface. In a preferred embodiment of the invention at least one of the microneedles or microcables senses the cellular electrical activity and harvests cellular energy and at least one of the microneedles or microcables of the tissue interface is used for emitting an electrical pulse if needed.

In one embodiment of the invention at least one microneedle or microcable of the tissue interface used to emit an electrical pulse is set by programming during implantation of the cardiac pacemaker by the external programmer unit. In a further embodiment of the invention, at least one microneedle or microcable of the tissue interface used to emit an electrical pulse is set by programming any time after the implantation by the external programmer unit.

In a preferred embodiment those microneedle or microneedles or microcable or microcables are selected to emit an electrical pulse if needed which has/have the lowest pace-threshold. Every microneedle or microcable has its own pace-threshold. The pace-threshold is defined as the energy needed to activate the tissue. Pace-thresholds are defined by the external programmer unit based on the sensed amplitude of the cellular electrical activity. This is performed automatically on the chip or by the external programmer unit.

In a further embodiment a microneedle or microcable is automatically switched from pacing to sensing/harvesting after the full stimulation pulse has been deployed. The functions just have a different time allocation on the microneedle or microcable.

In one embodiment the cardiac pacemaker is adapted to undertake an algorithm-based combination and/or comparison of sensed cellular electrical activity from multiple microneedles or microcables. This is useful to assess an automatic reference level and pace-threshold or for an automatized optimization of microneedle function adjudication. Due to the heart activity during one heart cycle the amplitude of the cellular electrical activity reaches the reference level, rises and falls again below the reference level. In case of normal heart activity this process is repeated for every heartbeat. After a certain time after the sensed amplitude of the cellular electrical activity has exceeded the reference level and has been fallen again below the reference level it is expected that the sensed amplitude of cellular electrical activity exceeds again the reference level due to the next cardiac event.

According to the invention, if the cellular electrical activity exceeds the reference level within a cardiac event, a time window starts, which is called cardiac cycle time. It is expected that inside a given cardiac cycle time the sensed cellular electrical activity falls below the reference levels and rises again until it exceeds the reference level due to the start of the following cardiac cycle. If no cellular electrical activity is sensed above the reference level on all microneedles or microcables, which is above the respective reference level of the microneedles or microcables, an electrical pulse is emitted into the tissue at the end of the cardiac cycle time. According to the invention the cardiac cycle time is between 4000 ms and 1500 ms, preferably the cardiac cycle time is 1000 ms. The cardiac cycle time is set via the external programmer unit after implantation of the cardiac pacemaker and can be adjusted any time via the external programmer unit.

The cardiac cycle time starts over again every time the sensed amplitude of the cellular electrical activity reaches the reference level and rises further. Further, the cardiac cycle time starts over again after emitting an electrical pulse into the myocardial tissue. With the cardiac cycle time it is monitored if cardiac events appear in the expected time windows.

As already described the heartbeat increases upon physical activity. Thus, the cardiac cycle length should be decreased if the patient is physically active. In one embodiment of the invention, physical activity of a patient is detected by a sensor and the sensor gives the command to the chip to set the cardiac cycle length to a minimum cardiac cycle length. Therefore, a minimum cardiac cycle length is defined which is programmable to the chip via the external programmer unit. In one embodiment the minimum cardiac cycle length is between 400 ms and 650 ms. Preferably the minimum cardiac cycle length is set via the external programmer unit.

According to the invention, the cardiac pacemaker comprises safety features. In one embodiment of the invention no electrical pulse is emitted as long as at least one of the microneedles or microcable senses a cellular electrical activity above the reference level of the corresponding microneedle or microcable. Thereby, it is omitted that an electrical pulse is emitted into an active heartbeat.

Further in one embodiment of the invention a safety margin is comprised which starts after the cellular electrical activity falls below the reference level. During the safety margin no electrical pulse is emitted. The safety margin is between 50 ms and 100 ms and guarantees that no electrical pulse is emitted in the vulnerable period, in which heart rhythm disturbances can be induced.

However, in case of cardiac cycles in which less than 10% of all microneedles or microcables of the tissue interface sense cellular electrical activity with an amplitude less than 10% above reference level uncertainty arises, whether there is true cellular electrical activity or e.g. if the signals are artifacts or arise from misleading external fields. To prevent a failing heartbeat in such cases, in one embodiment of the invention an electrical pulse is emitted 100 ms after the reference level of the cellular electrical activity was reached if the above-mentioned conditions are fulfilled. This can be programmed by the external programmer unit to adapt the safety feature to individual patient's needs.

Advantageously, in a preferred embodiment, a microneedle or microcable which is programmed to emit an electrical pulse if needed, can also sense cellular electrical activity and harvest energy in a cardiac cycle, when there is cellular electrical activity.

Each of the microneedles or microcables of the tissue interface represents a separate electrical connection into a cardiac tissue if the cardiac pacemaker is implanted in a patient, wherein each of the microneedles or microcables is individually programmable by the external programmer unit. Thereby, the cardiac pacemaker comprises more electrical connections into a cardiac tissue than any other device available today. Accordingly, a redundancy of electrical connections is provided by the cardiac pacemaker according to the invention. In contrast, state-of-the-art cardiac pacemakers have only one electrical connection, in form of a wire. If that fails due to electrode problems or degradation of the electrical electrode/tissue interface (e.g. through aging and fibrosis), patients need an operation and a new wire.

The redundancy of the microneedles ensures continuous cardiac pacemaker functionality even in case of degeneration of a single microneedle or microcable/tissue interface. If one microneedle or microcable degrades over time for whatever reason its function can be taken over by another microneedle or microcable with a better functionality.

Advantageously, the microneedles or microcables functionality can be exchanged or replaced through configuration reprogramming throughout the entire device lifetime.

Further, due to the improved programmability of sensing and pacing due by programming options the energy consumption of the cardiac pacemaker according to the invention is less compared to the energy consumption of cardiac pacemaker of the state-of-the-art.

The cardiac pacemaker according to the invention provides an improved interpretability of electrical signals in the heart due to instant comparison of hundreds of independent electrical recordings. Moreover, an improved automatized threshold testing due to comparability of signals between neighboring microneedles or microcables of the tissue interface is provided, where one is stimulating and the other one recording. Further, more targeted selection of cardiac pacing sites, e.g. pacing into the specific conduction system (His bundle pacing) is provided due to the availability of multiple anatomically redundant different spatial pacing locations.

### Method for stimulating myocardial tissue

Further, the present invention provides a method for stimulating myocardial tissue utilizing a cardiac pacemaker according to the invention, characterized in that
- the microneedles or microcables of the tissue interface are inserted into myocardial tissue;
- a cardiac cycle time is set;
- optionally a minimum cardiac cycle time is set;
- at least one reference level for the cellular electrical activity is set;
- at least one microneedle or microcable is set to emit a monophasic electrical pulse;
- at least one microneedle or microcable is set to sense the amplitude of the cellular electrical activity and to harvest energy;
- the amplitude of the cellular electrical activity is sensed and energy is harvested at least by one microneedle or microcable;
- the cardiac cycle time starts if the amplitude of the cellular electrical activity sensed by at least one microneedle or microcable reaches the reference level of the corresponding microneedle or microcable a monophasic electrical pulse is emitted into the myocardial tissue by at least one microneedle or microcable; and
- an electrical pulse is applied to the myocardial tissue by at least one microneedle or microcable if no cellular electrical activity with an amplitude above the reference level is sensed anymore during the cardiac cycle time after the amplitude of the sensed cellular electrical activity has been fallen below the reference level;

wherein the electrical pulse is generated utilizing the harvested energy; and
wherein energy is harvested by at least one microneedle or microcable immediately after emitting the monophasic pulse.

All features described above also apply for the method for stimulating myocardial tissue and vice versa.

According to the method of the invention the distal ends of the microneedles or microcables of the tissue interface are inserted into myocardial tissue. The penetration depth of the microneedles of the array of microneedles is between 1 mm and 15 mm.

In one embodiment the cardiac pacemaker can be fixated by fixation devices through the fixation holes in the interposer layer which are outside the lid. In a further embodiment the cardiac pacemaker can be fixated by helix screws, anchors or hooks. In one embodiment the cardiac pacemaker is fixated by the microneedles or microcables of the tissue interface, especially if they have a helix shape.

In one embodiment of the invention the cardiac pacemaker is deployed through a catheter, which is advanced from a femoral venous puncture site into the heart as already described above in more detail.

According to the invention, a cardiac cycle length, optionally a minimum cardiac cycle length and a reference level for the cellular electrical activity are set by the external programmer unit. Further, at least one microneedle or microcable is set to emit an electrical pulse and at least one microneedle or microcable is set to sense cellular electrical activity and to harvest energy. This is also done via the external programmer unit. These steps can be done immediately after the implantation of the cardiac pacemaker. Advantageously, adjustments of these parameters can be done any time via the external programmer unit.

Preferably, a minimum cardiac cycle length is set if the cardiac pacemaker according to the invention comprises at least one sensor, wherein the sensor is an activity sensor.

According to the method, the amplitude of the cellular electrical energy is sensed, and energy is harvested at least by one microneedle or microcable. The harvested energy is collected into the at least one buffer capacitor. In one embodiment of the invention the harvested energy is collected into multiple buffer capacitors, wherein the multiple buffer capacitors constitute a buffer capacitor-array.

Further, an electrical pulse is applied to the myocardial tissue by at least one microneedle or microcable of the tissue interface if no cellular electrical activity is sensed anymore during the cardiac cycle time with an amplitude above the reference level after the sensed amplitude of the cellular electrical activity has been fallen below the reference level once. Preferably, the electrical pulse is applied to the myocardial tissue by the microneedle or microcable having the lowest energy demand. The microneedle or microcable with the lowest energy demand is determined by the amplitude of the cellular electrical energy sensed by the microneedles or microcables of the tissue interface.

In a further embodiment of the invention several microneedles or microcables are set to emit an electrical pulse. In one embodiment between 1 and 40 microneedles or microcables of 100 microneedles or microcables, preferably between 1 and 30 microneedles or microcables of 100 microneedles or microcables, most preferably 1 microneedle or microcable of 100 microneedles or microcables is set to emit an electrical pulse.

In one embodiment more than one microneedle or microcable is set to sense and harvest cellular electrical energy. Accordingly, in one embodiment all microneedles or microcables which are not set to emit an electrical pulse are set to sense and harvest cellular electrical energy.

The electrical pulse which is applied to the myocardial tissue is monophasic as commonly used to stimulate cardiac tissue with cardiac pacemakers.

In one embodiment for pacing and sensing recordings between two microneedles or microcables are done. This gives different signals with different information and maybe other pacing thresholds.

In one embodiment of the invention the motion of a patient wearing the cardiac pacemaker is sensed by a sensor. This information can be used to adjust the cardiac cycle time of the cardiac pacemaker to the metabolic needs associated with the physical activity of a patient. This is done by setting the cardiac cycle time to the minimum cardiac cycle time. Accordingly, an electrical pulse is applied to the myocardial tissue by at least one microneedle or microcable of the tissue interface if no cellular electrical activity is sensed anymore during the minimum cardiac cycle time with an amplitude above the reference level after the sensed amplitude of the cellular electrical activity has been fallen below the reference level once.

According to the invention energy is harvested by at least one microneedle or microcable immediately after emitting the monophasic pulse. In one embodiment energy is harvested by the same microneedle or microcable which has emitted the pulse immediately after emitting the monophasic pulse.

State-of-the-art clinical pacemakers use a stimulation waveform which has two components. Each component has a distinctly different biological "purpose". The first component is the "monophasic pace pulse". Its purpose is to depolarizes cells/tissue above the membrane threshold, beyond which the cells autonomously create Action Potentials. It triggers the self-discharge of cells. Then the cellular excitation autonomously spreads to neighboring cells and across the organ. This phase has an excitation/depolarization purpose. It requires active energy injection. The pacing threshold (PT) which is measured in clinical practice, characterizes voltage and duration of this first component (= the monophasic pace pulse). It describes voltage and duration needed to effectively excite (depolarize) the cardiac tissue to start its own Action Potentials.

The second component is the "recharge phase". It is a passive current flow back into the pacemaker. Its purpose is to balance charge at the metal-electrolyte interface. It recovers the charge, that was deployed during the "monophasic pace pulse" and which is stored in the capacitances of the metal-electrolyte interface and inside the safety coupling cap. This charge is inversely collected and wasted as micro-heat. It has a much lower voltage with a much longer duration. In the end both components of the waveform together create nearly charge equilibrium.

With the present invention, charge neutrality is achieved by switching on the harvester immediately after the monophasic pacing pulse. Contrary to existing clinical pacemakers, where the charge neutrality phase ("recharge phase") wastes the recovered energy, the present invention harvests (and re-utilizes) this energy out of the capacitances of the metal-electrolyte interface and the safety coupling cap.

The present invention provides a high flow of energy out of the tissue source into the harvester. Several aspects of the tissue interface and its interaction with the tissue have helped to achieve this result.

Firstly, a dedicated connection of the harvesting microneedles or microcables to the intramyocardial extracellular tissue compartment is guaranteed. Further advantageously electrical compartmentalization of harvesting and reference microneedle or microcable locations with discrimination between epicardium, myocardium, endocardium, and blood pool is realized. This drives the design and helps to sustainably record harvestable electrograms (monophasic action potentials) by preventing current shunting between compartments, hence the harvesting and reference electrodes.

Further, fractal Titanium Nitride (TiN) coating is partially deposited on the microneedles or microcables of the inventive tissue interface which increases the electrochemical surface, favorably lowers source impedances, increases harvesting currents, and improve energy transfer.

In one embodiment the harvested energy is collected into the at least one buffer capacitor, a buffer capacitor-array, or an existing standard electrochemical battery.

In one embodiment motions of the biological tissue are sensed by a sensor and the cardiac cycle length is set to the minimum cardiac cycle length if motions of the biological tissue are sensed.

The invention is further described by 9 figures.
- Figure 1: illustrates an electrical model of the tissue interface
- Figure 2: illustrates a cross section of a tissue interface;
- Figure 3: (A) illustrates the accumulated harvested energy from a single microneedle over time in an animal study; (B) illustrates the signal of a single microneedle over time;
- Figure 4: illustrates one embodiment of a microneedle;
- Figure 5: (A) and (B) illustrate microcables according to the invention;
- Figure 6: (A) to (C) illustrate cardiac pacemakers according to the invention;
- Figure 7: (A) and (B) illustrates one embodiment of the cardiac pacemaker;
- Figure 8: illustrates a cardiac pacemaker inside the heart;
- Figure 9: illustrates the pacing waveform of a state-of-the-art cardiac pacemaker.

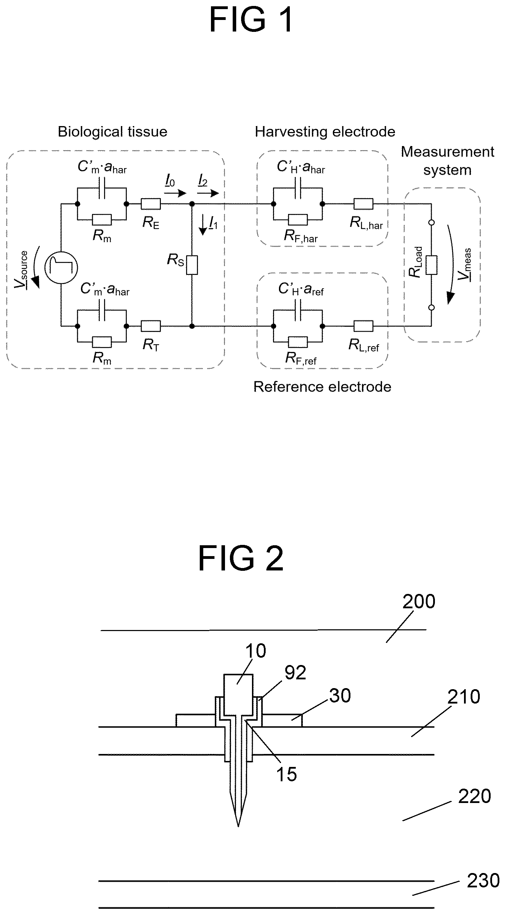

**Figure 1** illustrates an electrical model of the tissue interface with four main components: (i) measurement system, (ii) harvesting microneedle inside the myocardial tissue, (iii) reference microneedle in the blood pool, and (iv) biological tissue as an voltage source.

**Figure 2** illustrates a cross section of the tissue interface with one microneedle 10. The microneedle 10 is fixed on an interposer layer 30 and penetrates the tissue. Exemplarily the tissue is a heart wall. The microneedle 10 penetrates the endocardium 210 and ends in the myocardium 220. The interposer layer is positioned in the intracardial blood pool 200. The epicardium 230 is not penetrated. The microneedle 10 comprises a coating 15 and an isolation 92, wherein the isolation 92 covers the microneedle 10 in the tissue compartment of the endocardium 210 but not in the tissue compartment of the myocardium 220.

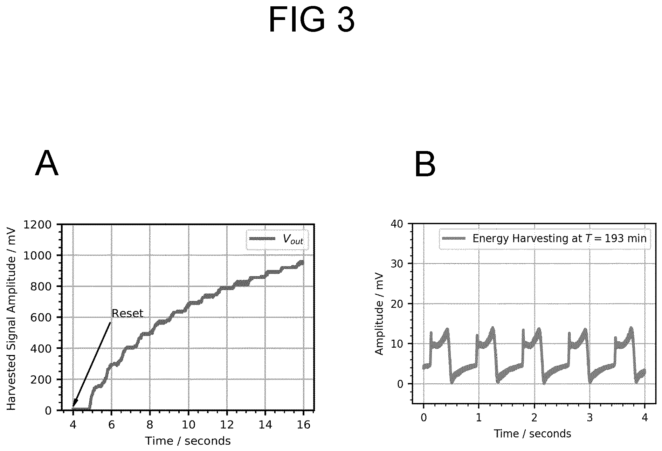

**Figure 3A** illustrates a single microneedle charge transfer, charge accumulation, and voltage conversion. The harvester output voltage (Vₒᵤₜ) was measured across capacitor Cout using an isolated oscilloscope.

**Figure 3B** illustrates a signal of a single microneedle during a late instance of energy harvesting, at T=3h 13minutes. The signal is loaded with the 1kOhm source impedance of the energy harvester. (normalized baseline DC offset of -6.5 mV)

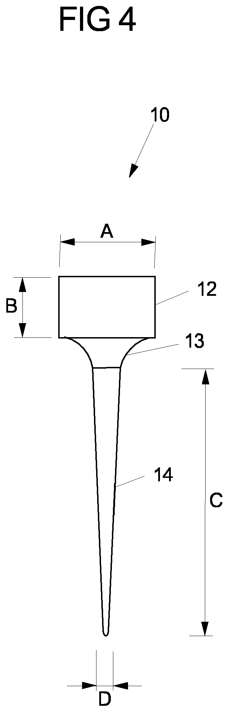

**Figure 4** illustrates one embodiment of a microneedle 10. The microneedle 10 comprises a proximal end 12, a tapered portion 13 and a distal end 14, wherein the tapered portion 13 connects the proximal end 12 with the distal end 14. Accordingly, the tapered portion 13 is as short as possible and serves only as connection between the proximal end 12 and the distal end 14. In one embodiment of the invention, the microneedle 10 according to the invention has a proximal end 12, which is shaped cylindrical with a diameter A between 0.05 mm and 0.5 mm, preferably the proximal end has a diameter A of 0.1 mm and a height B between 0.05 mm and 0.5 mm, preferably with a height B of 0.2 mm. The distal end 14 is needle shaped and has a length C between 0.5 mm and 10.0 mm. The distal end 14 of the microneedle 10 is electrically conductive and shear stress resistant in the range of 5 to 50 Newton, which is comparable to the shear stress resistance of bonding wires. Preferably the microneedle is milled from one piece. Preferably, the diameter D of the distal end 14 of the microneedle is between 0.001 mm and 0.1 mm, preferably between 0.01 mm and 0.1 mm, most preferably the diameters D of the distal end 14 of the microneedle is 0.02 mm. Thereby, the distal end 14 of the microneedle 10 approximates myocardial cellular dimensions.

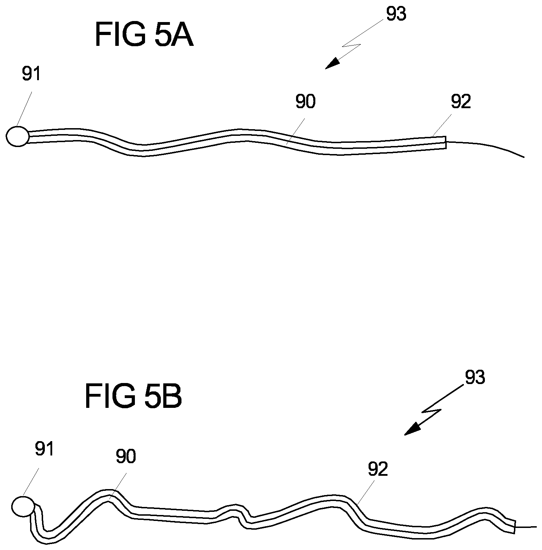

**Figure 5** illustrate microcables (filaments) 93 according to the invention. The microcables (filaments) 93 comprise a wire 90 and can be connected to a capacitor or capacitor configuration Cₙ by a soldering bump or other means such as PCB (Printed Circuit Board) or AME (Additive Manufacturing of Electronics) or simple wired connections illustrated by 91. The microcables 93 are covered with an isolation 92.

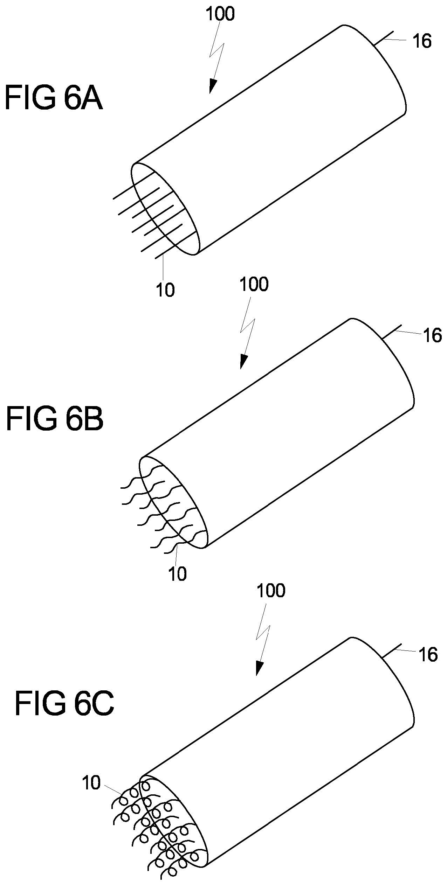

Figure 6 A to C illustrate an embodiments of the cardiac pacemaker 100 according to the invention having an cylindrical shape. Figure 6A illustrates a cardiac pacemaker 100 with microneedles 10 and a reference electrode 16. Figure 6B illustrates a cardiac pacemaker 100 with microcables 93 and a reference electrode 16. Figure 6C illustrates a cardiac pacemaker 100 with microneedles 10 which have the shape of helices.

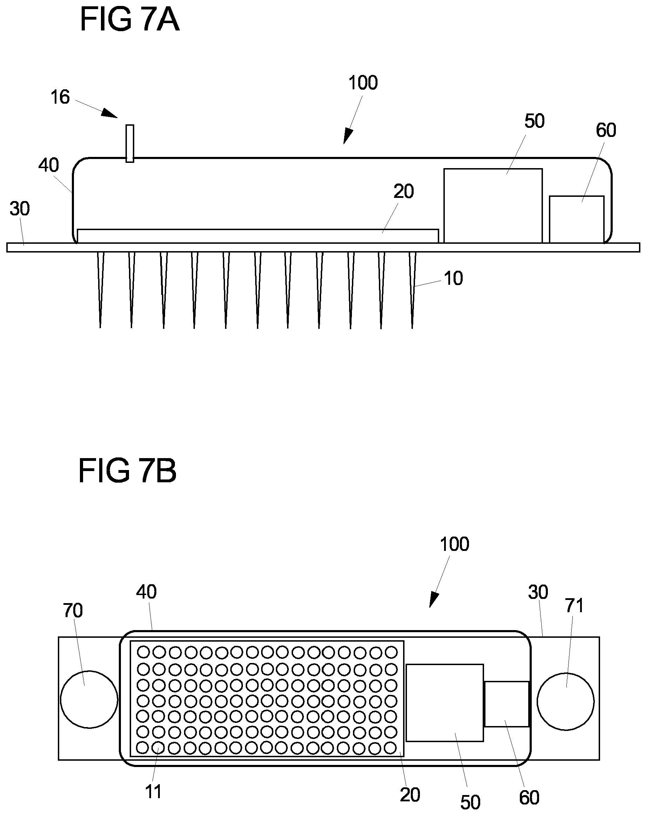

**Figure 7A** illustrates a further embodiment of the cardiac pacemaker 100 according to the invention in side view. On top of the interposer layer 30 the chip 20, a sensor 50 and a capacitor 60 are positioned. Furthermore, a coil is positioned on top of the interposer layer 30, which is not shown in the figure for the sake of clarity. The chip 20, the sensor 50, the capacitor 60 and the coil are covered by lid 40. The lid 40 covers the chip 20, the sensor 50 and the capacitor 60 from the surrounding, wherein the lid 40 is sealed to the interposer layer 30. Sealing can be done by adhesives or soldering tin. If adhesives are used the adhesive should be hardened. However, the sealing should be bio-compatible and insensitive to body fluids. Accordingly, the lid 40 and the interposer layer 30 form a capsule for the chip 20, the sensor 50 and the capacitor 60. The lid 40 and the interposer-layer 30 shield the electronic parts from surrounding body-fluids like blood, e.g. from body fluids by forming a capsule. The proximal end of each microneedle 10 is soldered to the surface of the chip 20, which ensures that each microneedle 10 of the array of microneedles has a direct contact to the chip 20. According to the invention each microneedle 10 of the array of microneedles is isolated from each other microneedle 10 of the array of microneedles. Further, the distal end of every microneedle 10 protrudes from the chip 20 and the interposer layer 30. The cardiac pacemaker 100 has a reference electrode 16.

**Figure 7B** illustrates one embodiment of the cardiac pacemaker 100 according to the invention in top view. Again, the chip 20, the sensor 50, the capacitor 60 and the coil are positioned on the interposer layer 30 and covered by the lid 40. The proximal end of each microneedle 10 is soldered by soldering points 11 to the surface of the chip 20, which ensures that each microneedle 10 of the array of microneedles has a direct contact to the chip 20. Further the interposer layer 30 comprises two fixing holes 70, 71. Those fixing holes 70, 71 are positioned outside the lid 40 on each side of the lid 40 in the interposer layer 30. The fixing holes 70, 71 are suited to serve for the fixation of the cardiac pacemaker 100 into myocardial tissue. The cardiac pacemaker 100 can be fixated by screws, clamps or such like devices through the fixing holes 70, 71 in the interposer layer 30.

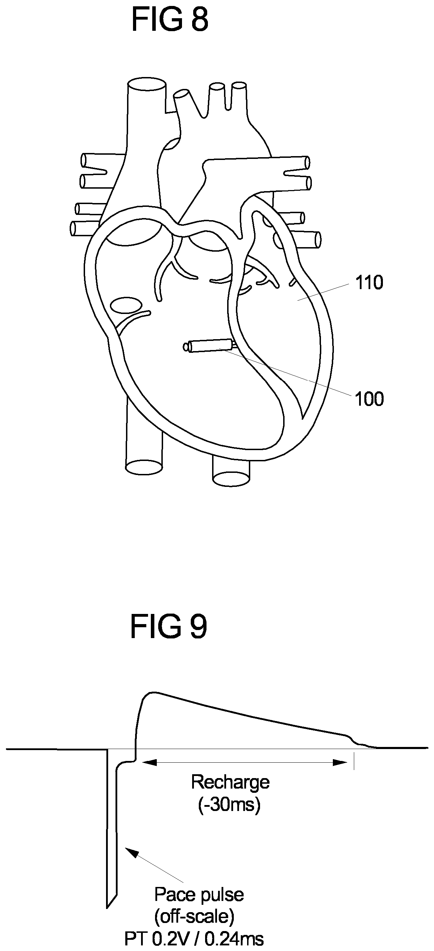

Figure 8 illustrates a cardiac pacemaker 100 according to the invention inside a heart chamber of a heart 110.

**Figure 9** illustrates a pulse with a biphasic waveform of a state-of-the-art clinical pacemaker, where each phase has a distinctly different biological "purpose", which is already described above in detail [8].

### References

[1] N. Franzina et al., "A miniaturized endocardial electromagnetic energy harvester for leadless cardiac pacemakers", PLoS ONE, 15(9): e0239667; https://doi.org/10.1371/journal.pone.0239667
[2] H. Ryu et al., "Self-rechargeable cardiac pacemaker system with triboelectric nanogenerators", Natire Communications 12, Article number 4374 (2021), https://doi.org/10.1038/s41467-021-24417-w
[3] D.H. Kim et al., "In Vivo Self-Powered Wireless Transmission Using Biocompatible Flexible Energy Harvester", Advanced Functional Materials, Vol. 27, Issue 24 (2017), https://doi.org/10.1002/adfm.201700341
[4] B. Shi et al., "A Packaged Self-Powered System with Universal Connectors Based on Hybridized Nanogenerators", Advanced Materials, Vol. 28, Issue 5 (2016), https://doi.org/10.1002/adma.201503356
[5] Mercier PP, Lysaght AC, Bandyopadhyay S et al., "Energy extraction from the biologic battery in the inner ear", Nat Biotechnol. 30, 1240-1243 (2012)
[6] Catacuzzeno L, Orfei F, Michele AD et al., "Energy harvesting from a bio cell", Nano Energy 56, 823-827 (2019)
[7] Clementi G, Neri I, Cottone F et al., "Biological Self-powered temperature sensors harnessing membrane potential of living cells", Nano Energy 121, doi.org/10.1016/j.nanoen.2023.109211 (2024)
[8] Zhu Q, Lang DJ, "Effect of pacemaker output coupling capacitor on intracardiac evoked response sensing", In Proceedings of the 20th Annual International Conference of the IEEE Engineering in Medicine and Biology Society, IEEE 20, 420-422 (1998)

### List of reference numerals

- 10: microneedle
- 11: soldering point
- 12: proximal end
- 13: tapered portion
- 14: distal end
- 15: coating
- 16: reference electrode
- 20: chip
- 21: wire
- 30: interposer layer
- 31: hole
- 33: soldering point
- 40: lid
- 50: sensor
- 60: capacitor
- 70, 71: fixing hole
- 80: protective cover
- 90: wire
- 91: connection
- 92: isolation
- 93: microcable
- 100: cardiac pacemaker
- 110: heart
- 200: intracardial blood pool
- 210: endocardium
- 220: myocardium
- 230: epicardium

## Claims

1. Tissue interface which is adapted to contact with a voltage source inside a tissue comprising at least one microneedle or one microcable
**characterized in**
**that** a part of the at least one microneedle or the at least one microcable comprises an isolating layer; and
**that** a part of the at least one microneedle or at least one microcable comprises a coating.

2. Tissue interface according to claim 1, **characterized in that** the coating is selected from the group comprising Titanium Nitride.

3. Tissue interface according to one of the preceding claims, **characterized in that** the at least on microneedle or the at least one microcable comprises a material selected from stainless steel, Platin/Iridium (Ptlr), gold, fine metals, refractory metals, conductive organic materials and conductive plastics or is made from one of these materials.

4. Tissue interface according to one of the preceding claims, **characterized in that** the isolating layer comprises a material selected from the group comprising organic materials or is made from one of these materials.

5. Tissue interface according to one of the preceding claims, **characterized in that** the coating has a porous or fractal surface.

6. Tissue interface according to one of the preceding claims, **characterized in that** a part of the at least one microneedle or the at least one microcable has the structure of a helix or a 3D ball or 3D cage.

7. Tissue interface according to one of the preceding claims, **characterized in that** the tissue interface comprises an array of 1 to 10000, preferably 2 to 1000, most preferably 10 to 250 microneedles or microcables.

8. Tissue interface according to claim 7, **characterized in that** at least the length of at least one microneedle or microcable differs from the length of the other microneedles or microcables.

9. Tissue interface according to any of the preceding claims, **characterized in that** cell potentials from living cells are used as voltage source.

10. A cardiac pacemaker, **characterized in that** the cardiac pacemaker comprises
a tissue interface according to claim 7;
at least one reference electrode;
a chip comprising at least one comparator with adaptive level, a sequence control circuit, at least one capacitor stack built by n capacitors and 2n switches, at least one buffer capacitor outside the at least one capacitor stack, at least two additional switches outside the at least one capacitor stack and a CMOS-Logic, wherein *n* ∈ *N* and *N* being positive integers *N* = {1,2,3, ...};
an interposer layer comprising holes for the microneedles or microcables of the tissue interface;
a lid;
at least one coil;
wherein the chip, is located on one surface of the interposer layer;
wherein the lid and the interposer layer form a capsule for the chip and the at least one coil;
wherein each microneedle or microcable has a distal end which protrudes from the chip; and
wherein the cardiac pacemaker is adapted to be electrically self-sufficient due to harvesting of electrical energy from myocardial cells.

11. Cardiac pacemaker according to claim 10, **characterized in that** the cardiac pacemaker is configured to record and store bioelectrical signals.

12. Method for stimulating myocardial tissue utilizing a cardiac pacemaker according to claim 10 or 11, **characterized in that**
• the microneedles or microcables are inserted into myocardial tissue;
• a cardiac cycle time is set;
• optionally a minimum cardiac cycle time is set;
• at least one reference level for the cellular electrical activity is set;
• at least one microneedle or microcable is set to emit a monophasic electrical pulse;
• at least one microneedle or microcable is set to sense the amplitude of the cellular electrical activity and to harvest energy;
• the amplitude of the cellular electrical activity is sensed and energy is harvested at least by one microneedle or microcable;
• the cardiac cycle time starts if the amplitude of the cellular electrical activity sensed by at least one microneedle or microcable reaches the reference level of the corresponding microneedle or microcable a monophasic electrical pulse is emitted into the myocardial tissue by at least one microneedle or microcable; and
• an electrical pulse is applied to the myocardial tissue by at least one microneedle or microcable if no cellular electrical activity with an amplitude above the reference level is sensed anymore during the cardiac cycle time after the amplitude of the sensed cellular electrical activity has been fallen below the reference level;
wherein the electrical pulse is generated utilizing the harvested energy; and
wherein energy is harvested by at least one microneedle or microcable immediately after emitting the monophasic pulse.

13. Method according to claims 12, **characterized in that** the harvested energy is collected into the at least one buffer capacitor, a buffer capacitor-array, or an existing standard electrochemical battery.

14. Method according to any of claims 12 or 13, **characterized in that** motions of the biological tissue are sensed by the sensor and that the cardiac cycle length is set to the minimum cardiac cycle length if motions of the biological tissue are sensed.
